# EUROPÄISCHE PATENTSCHRIFT

(11) **EP 2 015 712 B1**
(45) Veröffentlichungstag und Bekanntmachung des Hinweises auf die Patenterteilung: **11.08.2010**
(21) Anmeldenummer: 07722393.1
(22) Anmeldetag: 08.05.2007
(51) Int. Cl.: A61F 2/64, A61F 2/68

(54) **STEUERUNG EINES PASSIVEN PROTHESENKNIEGELENKES MIT VERSTELLBARER DÄMPFUNG**
CONTROL OF A PASSIVE PROSTHETIC KNEE JOINT WITH ADJUSTABLE DAMPING
SYSTÈME DE COMMANDE D'UNE PROTHÈSE PASSIVE DE L'ARTICULATION DU GENOU À AMORTISSEMENT RÉGLABLE

(30) Priorität: 09.05.2006 DE 102006021802
(43) Veröffentlichungstag der Anmeldung: 21.01.2009
(73) Patentinhaber: Otto Bock Healthcare GmbH, 37115 Duderstadt (DE)
(72) Erfinder: PUSCH, Martin, 37115 Duderstadt (DE); ZARLING, Sven, 37115 Duderstadt (DE); BOITEN, Herman, 37085 Göttingen (DE)
(74) Vertreter: Stornebel, Kai
(86) Internationale Anmeldenummer: PCT/DE2007/000841
(87) Internationale Veröffentlichungsnummer: WO 2007/128299

(56) Entgegenhaltungen:
- WO-A-2005/087144
- US-A1- 2005 015 156

## Beschreibung

Die Erfindung betrifft die Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß, die an dem Prothesenkniegelenk befestigt sind, an das Treppaufgehen.

Prothesenträger, die eine Knieprothese benötigen, müssen bei der Einstellung ihrer Prothese hinsichtlich der Flexions- und Extensionsdämpfung Kompromisse eingehen, da passive Prothesenkniegelenke nur für bestimmte Anwendungen optimiert sind, so dass signifikant abweichende Bewegungsmuster nicht oder nur außerordentlich erschwert möglich sind. So benötigt der Bewegungsablauf des Gehens in der Ebene, für den die Mehrzahl der passiven Prothesenkniegelenke mit Flexions- und Extensionsdämpfung ausgelegt sind, eine wesentlich andere Dämpfungscharakteristik als das Treppaufgehen. Daher erfolgt das Treppaufgehen mit den herkömmlichen Kniegelenksprothesen durch den Prothesenträger dergestalt, dass vor der Treppe stehend das gesunde Bein auf die erste Stufe angehoben und das kontralaterale Bein auf dieselbe Stufe nachgezogen wird. Eine Erhöhung der Gehgeschwindigkeit kann gegebenenfalls durch das Aufsetzen des gesunden Beines auf jede zweite Stufe erzielt werden, was jedoch sehr anstrengend ist.

Die US 2005/0015156 A1 beschreibt eine Protheseneinrichtung mit einem einstellbaren Widerstand in einem Prothesenkniegelenk, mit der es möglich sein soll, eine Wechselschrift beim Hinaufgehen von Stufen oder Neigungen zu ermöglichen oder zu erleichtern. Dies erfolgt durch eine Einrichtung zum Variieren der Widerstandskraft bei der Beugung oder Erstreckung des Beines. Der Grad der Widerstandskraft wird durch eine Einrichtung zur Erfassung einer Anterior-Posterior-Bewegung auf der Grundlage einer Oberschenkbewegung gesteuert. Durch eine Relativbewegung des Oberteils des Prothesekniegelenkes im Unterteil wird durch den Prothesenträger der Dämpfungswiderstand eingestellt. Die Steuer des Beugewiderstandes erfolgt willentlich gesteuert durch den Prothesenträger.

Aufgabe der vorliegenden Erfindung ist es, eine Steuerung für ein passives Kniegelenk bereitzustellen, mit dem ein alternierendes Treppensteigen für einen Prothesenträger möglich ist. Erfindungsgemäß wird diese Aufgabe durch eine Steuerung mit den Merkmalen des Anspruchs 1 gelöst. Vorteilhafte Ausgestaltungen und Weiterbildungen der Erfindung sind in den Unteransprüchen ausgeführt.

Bei herkömmlichen Kniegelenkprothesen, die zum Gehen in der Ebene ausgelegt sind, führt die notwendige niedrige Extensionsdämpfung des Prothesenkniegelenkes dazu, dass beim Treppaufgehen eine schlagartige Extension beim Hochdrücken und damit eine unakzeptabel hohe Stoßbelastung des Prothesenträgers erfolgt. Auch ist ein Ausbalancieren beim Hochdrücken des mit der Prothese versehenen Beines nicht möglich, weil drei Gelenke, nämlich das Fußgelenk, das Kniegelenk und die Hüfte, übereinander stehen und der Prothesenträger nur das Hüftgelenk direkt ansteuern kann. Bereits beim Anheben des Prothesenfußes zum Erreichen der nächsthöheren Treppenstufe tritt das Problem auf, dass der Prothesenfuß an die Setzstufe bzw. an die Unterkante der nächsthöheren Treppenstufe geführt wird, da die notwendige Flexionsdämpfung in der Schwungphasensteuerung für das Gehen in der Ebene ein Erreichen der Oberseite der nächsthöheren Treppenstufe nicht möglich macht. Der in aktiven Kniegelenken vorgesehene Flexionsantrieb zum Anheben des Fußes und Extensionsantrieb beim Durchdrücken des Knies und Anheben des Körpers über das mit der Prothese versehene Bein ist sehr aufwendig und sehr schwer. Darüber hinaus ist die Schwungphasensteuerung für das Gehen in der Ebene bei diesen aktiven Prothesenkniegelenken stark eingeschränkt.

Die erfindungsgemäße Steuerung sieht vor, dass das passive Prothesenkniegelenk mit verstellbarer Dämpfung in Flexionsrichtung zur Anpassung einer Protheseneinrichtung mit einem oberseitigen Anschlussmittel und einem Verbindungselement zu einem Kunstfuß, die an dem Prothesenkniegelenk befestigt sind, an das Treppaufgehen zunächst das Detektieren eines momentenarmen Anhebens des Prothesenfußes vorsieht. Nach der Detektion eines momentenarmen Anhebens des Prothesenfußes wird die Flexionsdämpfung in der Anhebephase abgesenkt und zwar auf unterhalb eines Niveaus, das für ein Gehen in der Ebene geeignet bzw. optimiert ist. Durch die Absenkung des Flexionswiderstandes, indem die Flexionsdämpfung abgesenkt wird, ist es möglich einen Kniewinkel bei dem Anheben des Prothesenfußes zu erreichen, der das Betreten des Prothesenfußes auf der nächsthöheren Stufe ermöglicht. Bei einer Hüftflexion und einem momentenarmen Anheben des Prothesenfußes kann aufgrund der Massenträgheit des Prothesenfußes ein Kniewinkel bei einem passiven Prothesenkniegelenk ermöglicht werden, der bei einem Vorbringen der Hüfte oder bei einer entsprechenden Extension durch die Schwerkraft ausreicht, um die Treppenkante zu überwinden und den Prothesenfuß über der Treppenstufe zu positionieren. Dabei ist es vorteilhaft die Masseverteilung in der Prothese so zu gestalten, dass der Masseschwerpunkt möglichst distal angeordnet ist, beispielsweise im Verbindungselement zum Prothesenfuß oder im Prothesenfuß selbst. Dazu kann beispielsweise die Steuereinheit des Kniesystems distal statt knienah angeordnet werden, so dass ohne eine Gewichtszunahme der Prothese durch Extragewichte im Prothesenfuß der gewünschte Effekt der Knieflexion bei einem momentenarmen Anheben des Prothesenfußes erreicht wird.

Nach dem Absenken der Flexionsdämpfung wird während einer Aufsetz- und Hüftstreckphase, in der das mit der Prothese versehene Bein gestreckt wird, die Flexionsdämpfung und ggf. die Extensionsdämpfung auf ein Niveau oberhalb einer Dämpfung einer Schwungphasensteuerung für das Gehen in der Ebene angehoben, so dass eine kontrollierte Extension bzw. Streckung sowohl des Hüftgelenkes als auch das Kniegelenkes und des Fußgelenkes erfolgen kann.

Nach der Überwindung der Treppenkante wird das Knie durch die Schwerkraft gestreckt. Um eine Positionierung des über der Treppenstufe befindlichen Prothesenfußes zu ermöglichen, wird die Flexionsdämpfung vor der Streckung des Prothesenkniegelenkes erhöht, so dass die Positionierung des Prothesefußes durch den vom Patienten direkt kontrollierbaren Hüftwinkel erfolgen kann.

Die Flexionsdämpfung wird in der Aufsetz- und ggf. Hüftstreckphase bevorzugt auf einen maximalen Wert erhöht, um ein Absenken bei einem nicht ausreichenden Hüftstreckmoment zu vermindern oder zu vermeiden. Dabei ist es vorgesehen, dass die Flexionsdämpfung in der Aufsetz- und Hüftstreckphase bis zur vollständigen Hüftstreckung beibehalten wird.

Zur erleichterten Positionierung des Prothesenfußes ist es vorgesehen, dass eine Extensionsdämpfung während der Anhebe- sowie Aufsetz- und Hüftstreckphase eingestellt wird, um während der Anhebephase ein durch die Schwerkraft bedingtes Strecken des Prothesenkniegelenkes bzw. ein Herunterfallen des Prothesenfußes zu vermeiden und um während der Aufsetz- und Hüftstreckphase den Fuß kontrolliert aufsetzen zu können. Würde eine Extensionsdämpfung während der Hüftstreckphase vollständig unterbleiben, würde ein unnatürliches Nachobenschnellen des Patienten erfolgen, was zu einem Anschlag und einer abrupten Unterbrechung in maximaler Extension des Kniegelenkes führen würde. Ein Anheben des Prothesenfußes ohne Extensionsdämpfung kann bei offenen Treppen, also ohne Setzstufe, dazu führen, dass der Prothesenfuß unter die nächsthöhere Treppenstufe geschoben wird.

Die Flexionsdämpfung wird bevorzugt in Abhängigkeit von der Veränderung des Kniewinkels angehoben. Sobald ein festgelegter Kniewinkel erreicht wird, der in der Regel größer als ein Kniewinkel ist, der bei einer Schwungphasensteuerung für das Gehen in der Ebene geeignet ist, wird die Flexionsdämpfung angehoben. Alternativ oder ergänzend kann die Flexionsdämpfung in Abhängigkeit von der auf den Unterschenkelschaft einwirkenden Axialkraft angehoben oder abgesenkt werden. Sinkt die Axialkraft in einem ausreichend schnellen Maße bis annähernd 0 bei einem nahezu gestreckten Knie, ist dies ein Indikator für das Einleiten eines Treppaufgehvorgangs.

Ergänzend oder alternativ kann die Vertikalbeschleunigung des Beines, also Oberschenkel- oder Unterschenkel und Hüfte bei gleichzeitigem Axialkraftabfall aus Auslösung für das Aktivieren einer entsprechenden Flexionsdämpfungssteuerung und Extensionsdämpfungssteuerung für das Treppaufgehen erfolgen. Darüber hinaus kann eine ausreichend schnelle Hüftbeugung bei wenig oder fehlender Axialkraft eine Kniebeugung bewirken. Statt einer Axialkraft können Kniestreckmoment, Knöchelmoment oder eine Kombination der Kräfte und Momente erfasst werden, um den Treppaufgehmodus zu ermöglichen.

Das Detektieren eines momentenarmen Anhebens kann rein mechanisch über einen Taster oder über einen Kraft- oder Momentensensor erfolgen. Der Taster kann beispielsweise als ein in einer Führung gelagerter Schieber ausgebildet sein, der nur bei nahezu senkrechtem Anheben des Prothesenfußes in eine Schaltstellung verfährt, die die Flexionsdämpfung reduziert. Die Messung der Kräfte oder Momente kann durch bekannte Sensoreinrichtungen erfolgen. Alternativ kann das momentenarme Anheben über eine Messung der Horizontalbeschleunigung des Prothesenfußes und der Erfassung einer Beugung im Kniegelenk erfolgen. Bei einer geringen Horizontalbeschleunigung des Prothesenfußes, also bei einem nahezu senkrechten Anheben, erfolgt anders als beim Gehen in der Ebene eine hohe Beugung im Prothesenkniegelenk, was ein Treppaufgehen indiziert. Darüber hinaus kann eine Erfassung des Vorfußmomentes im Prothesenfuß erfolgen, um festzustellen, ob sich der Prothesennutzer während des Gehens in Horizontalrichtung bewegen möchte, was eine sehr hohe Vorfußbelastung im Prothesenfuß zur Folge hat, oder ob bei einem aufgesetzten Prothesenfuß eine Axialkraftverringerung sowie eine Flexion im Kniegelenk erfolgt.

Um die notwendige Flexion zur Überwindung der Stufenhöhe nach dem Anheben zu erreichen, kann eine Flexionsunterstützung in der Anhebephase über eine vorgespannte Feder oder einen anderen Kraftspeichermechanismus erfolgen. Ebenfalls kann eine freie Extension durch eine Feder unterstützt werden, wenn nach einer erfolgten Absenkung der Flexionsdämpfung eine gewisse Zeit vergangen ist. Dies ist aus Sicherheitsgründen erforderlich, um bei einem irrtümlichen Auslösen des Treppaufgehmodus nicht in eine ungewollte Dämpfungssteuerung zu verfallen.

Die Erhöhung der Flexions- und ggf. Extensionsdämpfung wird bevorzugt dann eingeleitet, wenn der Prothesenfuß nach dem Anheben wieder aufgesetzt wird, beispielsweise wenn eine Erhöhung der Axialkraft ermittelt wird. Alternativ kann bei einem annähernd konstant bleibenden Kniewinkel die Extensions- und Flexionsdämpfung angehoben werden.

Die Flexionsdämpfung kann in der Anhebephase auf einen minimalen Wert abgesenkt werden, so dass die in jedem System wirksame Dämpfung aufgrund von Reibung nicht weiter erhöht wird.

Das Detektieren sowohl des momentenarmen oder momentenfreien Anhebens als auch des Absenkens der Flexionsdämpfung kann mechanisch erfolgen, ebenso wie die Veränderung der verschiedenen Dämpfungen, um eine möglichst einfache Prothesenkonstruktion zu ermöglichen.

Nachfolgend wird ein Ausführungsbeispiel anhand der Figuren näher erläutert.

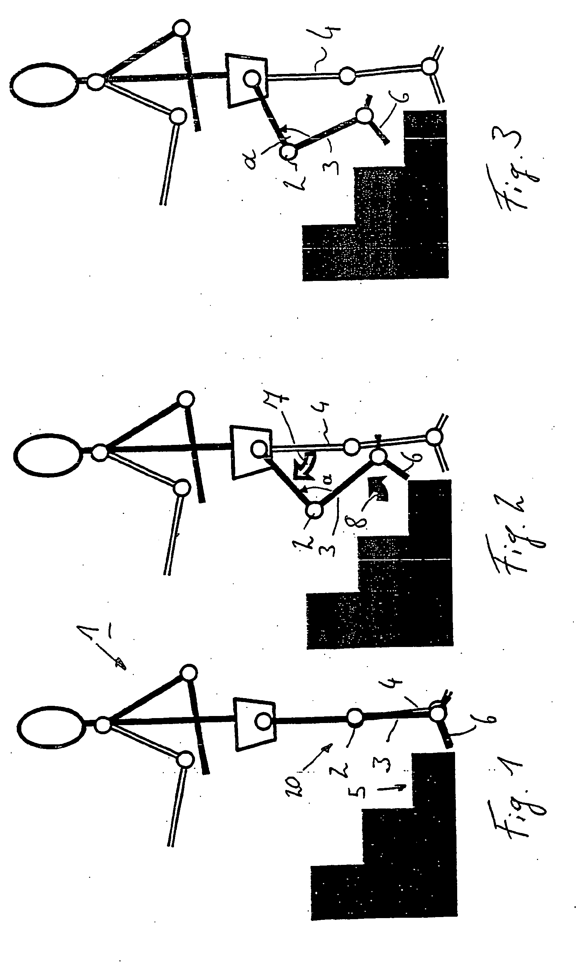

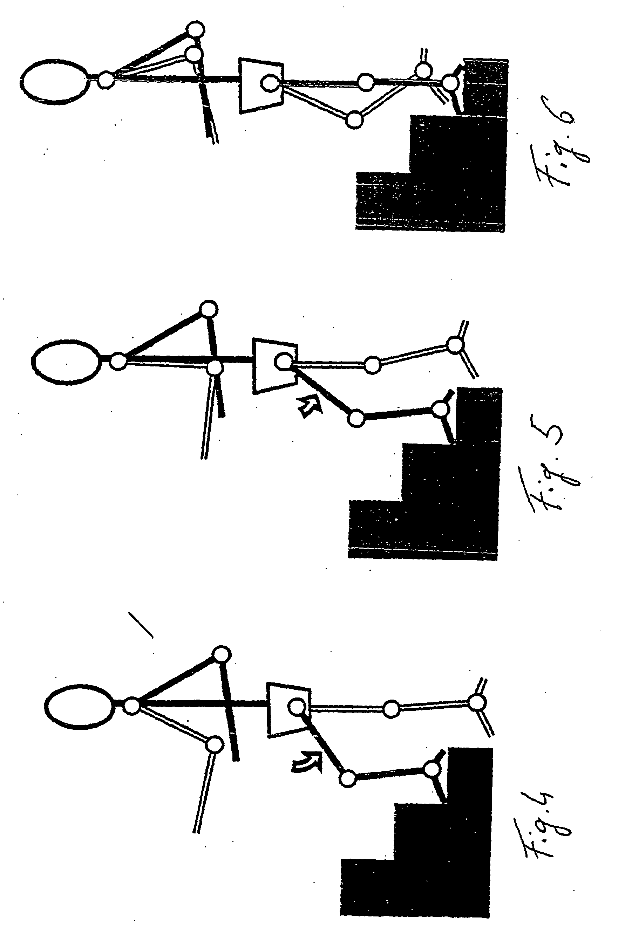

Die Figuren 1 bis 6 zeigen schematisch den Ablauf eines alternierenden Treppaufgehens mit passiver Kniegelenksprothese.

In der Figur 1 ist ein Prothesenträger 1 mit einer Kniegelenksprothese 2 dargestellt, die über oberseitige Anschlussmittel an einem Oberschenkelstumpf befestigt ist. Das Prothesenbein 20 steht mit dem gesunden kontralateralen Bein 4 vor einer Treppenstufe.

Zum Erreichen der nächsthöheren Stufe muss ein Prothesenfuß 6 um die Treppenkante geführt werden. Eine aktive Hüftbeugung, wie sie durch den Pfeil 7 angedeutet ist, unterstützt die passive Kniebeugung, die durch den Pfeil 8 gezeigt ist, die aufgrund der Massenträgheit sowohl des Prothesenfußes 6 als auch des Verbindungselementes 3 von dem Prothesenkniegelenk 2 zu dem Prothesenfuß 6 bewirkt wird. Hierzu ist eine minimale Flexionsdämpfung notwendig, damit nach einer Hüftflexion der Prothesenfuß 6 nicht nach vorne schwingt und gegen die Setzstufe oder unter die Treppenstufe 5 bewegt wird. Ziel ist es in der Abhebephase, wie sie in der Figur 2 dargestellt ist, den Prothesenfuß 6 möglichst senkrecht nach oben zu führen, gegebenenfalls eingeleitet durch eine leichte Bewegung nach hinten. Die Detektion des Anhebens erfolgt dabei über den Beugewinkel α zwischen dem Verbindungselement 3 und dem Oberschenkel oder über eine Verringerung der Axialkraft in dem Verbindungselement 3 ohne Flexion des Prothesenfußes 6. Ebenfalls ist es möglich, den Treppaufgehmodus und dadurch die Absenkung der Flexionsdämpfung auf einen Wert unterhalb der normalen Schwungphasensteuerung, bevorzugt auf den minimalen Wert, durch eine Horizontalbewegung des Prothesenfußes 6 nach hinten in Verbindung mit einer Hüftbeugung zu detektieren.

Nach dem Überwinden der Treppenkante und Beenden der Anhebephase, wie sie in der Figur 2 dargestellt ist, ist eine sichere Positionierung des Prothesenfußes 6 auf der Treppenstufe erforderlich. Dazu muss der Prothesenfuß 6 nach vorne bewegt werden, was durch Extension aufgrund der Schwerkraft erfolgen kann. Hierfür kann eine Extensionsdämpfung verringert werden, wenn dies nicht bereits in der Anhebephase erfolgt ist. Ein vor der Streckung in Flexion und Extension ausreichend bedämpftes Prothesenkniegelenk 2 ermöglicht die Positionierung des Prothesenfußes 6 durch den Prothesenträger 1, indem der Hüftwinkel verändert wird. In der Absenk- und Hüftstreckphase ist die Flexion und die Extension bevorzugt stark bedämpft, um neben der Kontrolle des Aufsetzens auch ein spontanes Zurückfallen bei einem nicht ausreichenden Hüftstreckmomentes zu verhindern. Die Extension bleibt gedämpft, um die Geschwindigkeit bei der Hüft- und Kniestreckung kontrollieren zu können. Dies ist in der Figur 3 dargestellt.

In der Figur 4 ist die Aufsetzphase abgeschlossen. Der Prothesenträger 1 kann mit einem Hüftstreckmoment die Kniestreckung einleiten. Die Kniestreckung kann durch eine Extension des gesunden Fußes unterstützt werden.

In der Figur 5 ist die zunehmende Kniestreckung durch das Aufbringen eines Hüftmomentes dargestellt. Die zunehmende Kniestreckung verkürzt den wirksamen Hebel und erleichtert die Kniestreckung durch die Hüftstreckung.

In der Figur 6 ist die vollständige Extension des mit der Kniegelenksprothese 2 versehenen Beines gezeigt. Das kontralaterale Bein 4 wird an dem Prothesenbein 20 vorbeigeführt und auf die nächst höhere Stufe gesetzt, so dass ein alternierendes Treppaufgehen mit passiver Kniegelenksprothese möglich ist.

Die Steuerung ist demnach so eingestellt, dass während des Anhebens des Prothesenfußes 6 ein Flexionswiderstand eingestellt wird, der einen Kniewinkel a erlaubt, der das Betreten des Prothesenfußes 6 auf der nächstfolgenden Stufe ermöglicht. Eine Flexionsunterstützung durch Federmechanismen können das Anheben und das Überwinden der Stufenhöhe erleichtern.

Sollte nach der Auslösung des Treppaufgehmodus durch Detektieren eines momentenarmen Anhebens keine Aktion folgen, wird eine freie Extension eingestellt, wobei die Einstellung der freien Extension zeitabhängig erfolgt. Das Zeitglied kann auch mechanisch erfolgen. Die Detektion des momentenarmen Anhebens kann über die Massenträgheit erfolgen, wenn das gesunde Bein zunächst aufgesetzt wird und erst die zweite Treppenstufe durch das mit der Prothese versehene Bein überwunden werden soll. Erfolgt zunächst das Entlasten des Prothesenfußes und dann ein Beugen im Prothesenkniegelenk, ist das Treppaufgehen einzustellen. Die Dämpfung sowohl in Extensionsrichtung als auch in Flexionsrichtung nach der Anhebephase, also während der Hüftstreckphase, wird beibehalten, bis eine vollständige Extension des Prothesenkniegelenkes erreicht bzw. detektiert ist.

## Patentansprüche

1. Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß, der an dem Prothesenkniegelenk befestigt ist, an das Treppaufgehen, **gekennzeichnet durch** folgende Schritte:
- Detektieren eines momentenarmen Anhebens des Prothesenfußes und
- Absenken der Flexionsdämpfung in einer Anhebephase auf unterhalb eines Niveaus, das für ein Gehen in der Ebene geeignet ist.

2. Steuerung nach Anspruch 1, **dadurch gekennzeichnet, dass** die Extensions und/oder Flexionsdämpfung in einer Aufsetz- und Hüftstreckphase auf ein Niveau oberhalb einer Dämpfung einer Schwungphasensteuerung für das Gehen in der Ebene angehoben wird.

3. Steuerung nach Anspruch 2, **dadurch gekennzeichnet, dass** die Flexionsdämpfung in der Aufsetzphase auf einen maximalen Wert erhöht wird.

4. Steuerung nach Anspruch 2 oder 3, **dadurch gekennzeichnet, dass** die Flexionsdämpfung in der Aufsetz- und Hüftstreckphase bis zur vollständigen Hüftstreckung beibehalten wird.

5. Steuerung nach einem der Ansprüche 2 bis 4, **dadurch gekennzeichnet, dass** die Flexionsdämpfung in Abhängigkeit von der Veränderung des Kniewinkels angehoben wird.

6. Steuerung nach einem der voranstehenden Ansprüche, **dadurch gekennzeichnet, dass** die Flexionsdämpfung in Abhängigkeit von der auf den Unterschenkelschaft einwirkenden Axialkraft angehoben oder abgesenkt wird.

7. Steuerung nach einem der voranstehenden Ansprüche, **dadurch gekennzeichnet, dass** eine Extensionsdämpfung während der Anhebe- sowie Aufsetz- und Hüftstreckphase eingestellt wird.

8. Steuerung nach einem der voranstehenden Ansprüche, **dadurch gekennzeichnet, dass** das Detektieren des momentenarmen Anhebens über einen Kraft- oder Momentensensor erfolgt.

9. Steuerung nach einem der voranstehenden Ansprüche, **dadurch gekennzeichnet, dass** das Detektieren des momentenarmen Anhebens über eine Messung einer Horizontalbeschleunigung des Prothesenfußes und Erfassung einer Beugung im Prothesenkniegelenk erfolgt.

10. Steuerung nach einem der voranstehenden Ansprüche, **dadurch gekennzeichnet, dass** die Detektion eines momentenarmen Anhebens über die Erfassung eines Vorfußmomentes im Prothesenfuß erfolgt.

11. Steuerung nach einem der voranstehenden Ansprüche, **dadurch gekennzeichnet, dass** eine Flexionsunterstützung in der Anhebephase über einen vorgespannten Federmechanismus erfolgt.

12. Steuerung nach einem der voranstehenden Ansprüche, **dadurch gekennzeichnet, dass** die Erhöhung der Flexions- und Extensionsdämpfung eingeleitet wird, wenn der Prothesenfuß nach dem Anheben wieder aufgesetzt wird.

13. Steuerung nach Anspruch 12, **dadurch gekennzeichnet, dass** das Aufsetzten über eine Axialkraftmessung in dem Unterschenkelschaft oder in dem Prothesenfuß detektiert wird.

14. Steuerung nach einem der voranstehenden Ansprüche, **dadurch gekennzeichnet, dass** die Flexionsdämpfung in der Anhebephase auf einen minimalen Wert abgesenkt wird.

15. Steuerung nach einem der voranstehenden Ansprüche, **dadurch gekennzeichnet, dass** nach erfolgter Absenkung der Flexionsdämpfung eine freie Extension zeitgesteuert eingestellt wird.

16. Steuerung nach Anspruch 15, **dadurch gekennzeichnet, dass** die freie Extension federunterstützt wird.

17. Steuerung nach Anspruch 15 oder 16, **dadurch gekennzeichnet, dass** die Zeitsteuerung mechanisch oder elektronisch erfolgt.

## Claims

1. A control of a passive prosthetic knee joint with adjustable damping in the direction of flexion such that a prosthetic unit, with upper attachment elements and with a connection element to an artificial foot, which is secured on the prosthetic knee joint, can be adapted for climbing stairs, **characterized by** the following steps:
- detecting a low-torque lift of the prosthetic foot, and
- lowering the flexion damping in a lift phase to below a level that is suitable for walking on level ground.

2. The control as claimed in claim 1, **characterized in that** the extension and/or flexion damping, in a foot placement and hip-straightening phase, is increased to a level above a damping of a swing phase control for walking on level ground.

3. The control as claimed in claim 2, **characterized in that** the flexion damping in the foot placement phase is increased to a maximum value.

4. The control as claimed in claim 2 or 3, **characterized in that** the flexion damping in the foot placement and hip-straightening phase is maintained until the hip is fully straightened.

5. The control as claimed in one of claims 2 through 4, **characterized in that** the flexion damping is increased as a function of the change of the knee angle.

6. The control as claimed in one of the preceding claims, **characterized in that** the flexion damping is increased or lowered as a function of the axial force acting on the lower leg shaft.

7. The control as claimed in one of the preceding claims, **characterized in that** an extension damping is set during the lift phase and also during the foot placement and hip-straightening phase.

8. The control as claimed in one of the preceding claims, **characterized in that** the low-torque lift is detected by a force or torque sensor.

9. The control as claimed in one of the preceding claims, **characterized in that** the low-torque lift is detected by measuring a horizontal acceleration of the prosthetic foot and by recording a bending in the prosthetic knee joint.

10. The control as claimed in one of the preceding claims, **characterized in that** a low-torque lift is detected by recording a torque at the front of the prosthetic foot.

11. The control as claimed in one of the preceding claims, **characterized in that** the flexion in the lift phase is supported via a pretensioned spring mechanism.

12. The control as claimed in one of the preceding claims, **characterized in that** the increase in the flexion and extension damping is initiated when the prosthetic foot, after being lifted, is placed down again.

13. The control as claimed in claim 12, **characterized in that** the foot placement is detected by an axial force measurement in the lower leg shaft or in the prosthetic foot.

14. The control as claimed in one of the preceding claims, **characterized in that** the flexion damping in the lift phase is lowered to a minimum value.

15. The control as claimed in one of the preceding claims, **characterized in that**, after the flexion damping has been lowered, a free extension is set with time control.

16. The control as claimed in claim 15, **characterized in that** the free extension is spring-assisted.

17. The control as claimed in claim 15 or 16, **characterized in that** the time control is effected mechanically or electronically.

## Revendications

1. Système de commande d'une prothèse passive d'articulation du genou comprenant un amortissement réglable dans la direction de flexion, pour adapter au monter d'escalier un dispositif de prothèse comprenant des moyens de raccordement situés du côté supérieur et un élément de liaison à un pied artificiel qui est fixé à la prothèse d'articulation de genou, **caractérisé par** les étapes suivantes :
- détection d'un soulèvement à moment réduit du pied artificiel et
- abaissement de l'amortissement de flexion dans une phase de soulèvement pour le ramener au-dessous d'un niveau qui est approprié pour une marche sur le plat.

2. Système de commande selon la revendication 1, **caractérisé en ce que** l'on augmente l'amortissement d'extension et/ou de flexion dans une phase de pose et d'étirement coxal, à un niveau supérieur à un amortissement d'un système de commande d'une phase de lancée pour la marche sur le plat.

3. Système de commande selon la revendication 2, **caractérisé en ce que** l'on augmente à une valeur maximale l'amortissement de flexion dans la phase de pose.

4. Système de commande selon la revendication 2 ou 3, **caractérisé en ce que** l'on conserve l'amortissement de flexion dans la phase de pose et d'étirement coxal jusqu'à l'étirement coxal complet.

5. Système de commande selon l'une des revendications 2 à 4, **caractérisé en ce que** l'on augmente l'amortissement de flexion en fonction de la variation de l'angle du genou.

6. Système de commande selon l'une des revendications précédentes, **caractérisé en ce que** l'on augmente ou l'on diminue l'amortissement de flexion en fonction de la force axiale s'exerçant sur la tige de bas de la jambe.

7. Système de commande selon l'une des revendications précédentes, **caractérisé en ce que** l'on règle un amortissement d'extention pendant la phase de soulèvement comme de pose et d'étirement coxal.

8. Système de commande selon l'une des revendications précédentes, **caractérisé en ce que** l'on effectue la détection du soulèvement à moment réduit par un capteur de moment ou de force.

9. Système de commande selon l'une des revendications précédentes, **caractérisé en ce que** l'on effectue la détection du soulèvement à moment réduit par une mesure d'une accélération horizontale de la prothèse de pied et détection d'un fléchissement dans la prothèse d'articulation de genou.

10. Système de commande selon l'une des revendications précédentes, **caractérisé en ce que** l'on effectue la détection d'un soulèvement à moment réduit par la détection d'un moment de l'avant-pied dans la prothèse de pied.

11. Système de commande selon l'une des revendications précédentes, **caractérisé en ce que** l'on effectue une assistance à la flexion dans la phase de soulèvement par un mécanisme à ressort précontraint.

12. Système de commande selon l'une des revendications précédentes, **caractérisé en ce que** l'on commence l'augmentation de l'amortissement de flexion et d'extension lorsque la prothèse de pied est de nouveau posée après le soulèvement.

13. Système de commande selon la revendication 12, **caractérisé en ce que** l'on détecte la pose par une mesure de force axiale dans la tige de bas de la jambe ou dans la prothèse de pied.

14. Système de commande selon l'une des revendications précédentes, **caractérisé en ce que** l'on diminue à une valeur minimale l'amortissement de flexion dans la phase de soulèvement.

15. Système de commande selon l'une des revendications précédentes, **caractérisé en ce que** l'on règle de façon commandée temporellement une extension libre après que la diminution de l'amortissement de flexion a eu lieu.

16. Système de commande selon la revendication 15, **caractérisé en ce que** l'extension libre est assistée par ressort.

17. Système de commande selon la revendication 15 ou 16, **caractérisé en ce que** la commande temporelle s'effectue mécaniquement ou électroniquement.
